(19) Europäisches Patentamt
European Patent Office
Office européen des brevets

(11) **EP 4 414 359 A1**

(12) **EUROPEAN PATENT APPLICATION**

(43) Date of publication:
**14.08.2024   Bulletin 2024/33**

(21) Application number: **24154384.2**

(22) Date of filing: **29.01.2024**

(51) International Patent Classification (IPC):
**C07C 231/06** (2006.01)   **C07C 233/05** (2006.01)
**C07C 233/09** (2006.01)   **C07C 233/11** (2006.01)
**C07C 233/65** (2006.01)   **C07D 213/00** (2006.01)

(52) Cooperative Patent Classification (CPC):
(C-Sets available)
**C07C 231/065; C07D 213/00**                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.01.2023   IT 202300001458**

(71) Applicant: **Università di Pisa**
**56126 Pisa (IT)**

(72) Inventors:
• **CIRRI, Damiano**
**56126 Pisa (IT)**
• **MARZO, Tiziano**
**56126 Pisa (IT)**
• **PRATESI, Alessandro**
**56126 Pisa (IT)**

(74) Representative: **Ceccarelli, Ilaria et al**
**Barzanò & Zanardo Roma S.p.A.**
**Via Piemonte, 26**
**00187 Roma (IT)**

(54) **CATALYTIC PROCESS FOR THE PREPARATION OF AMIDES**

(57)    The present invention relates to a palladium-arsenic catalytic process for the preparation of amides capable of advantageously converting a nitrile into the respective amide under mild reaction conditions, such as a neutral pH and low temperatures.

**FIG. 2**

EP 4 414 359 A1

(52) Cooperative Patent Classification (CPC): (Cont.)

C-Sets
**C07C 231/065, C07C 233/05;**
**C07C 231/065, C07C 233/09;**
**C07C 231/065, C07C 233/11;**
**C07C 231/065, C07C 233/65**

**Description**

The present invention relates to a catalytic process for the preparation of amides.

In greater detail, the invention relates to a palladium-arsenic catalytic process for the preparation of amides capable of advantageously converting a nitrile into the respective amide under mild reaction conditions, such as a neutral pH and low temperatures.

It is well known that the amide function represents one of the most important groups in various fields of chemistry. The peptide bond is indeed a type of amide bond that enables the performance of the key functions of proteins and, in turn, the chemistry of life [1].

Amides are "the fundamental building blocks" for several technological applications and organic, industrial, pharmaceutical and material chemistry applications. Amide groups are commonly found in high-value industrial chemical products, e.g. polymers, plasticisers, solvents [2] and lubricants [3]. For this reason, the search for increasingly efficient and convenient processes for the production of amides is of great interest to the chemical industry [4]. Several synthetic approaches can be used on a laboratory scale; however, most of them imply drastic experimental conditions that might not be tolerated by other functional groups present on the same molecule, or by the very amides that are generated [5]. Furthermore, the known methods typically suffer from a low atom economy, i.e. a low conversion efficiency of the specific chemical process, in relation to all the atoms involved in the process itself and the desired substances produced according to the equation shown below:

$$\text{Atom Economy} = (\text{Molecular Weight of the Desired Product} / \text{Molecular Weight of All the Products}) \times 100$$

Furthermore, the known methods are associated with the generation of large amounts of waste products; therefore, the abovementioned characteristics make these methods hardly sustainable [6]. In large-scale industrial production, the nitrile hydration process is undoubtedly a necessary choice due to the more favourable atom economy. Unfortunately, the latter approach as well has some significant drawbacks, since the hydration reaction is usually catalysed by strongly acidic or basic conditions that may result in the lack of good control over the reaction and, consequently, impair the stability of the amides themselves.

Moreover, due to the drastic reaction conditions, accurate control of the hydration process is often impracticable. In fact, on the one hand, in the case of base catalysis, the desired amide is often hydrolysed to the corresponding carboxylate with stoichiometric consumption of the catalyst. On the other hand, in the case of an acid-mediated process, careful control of the temperature and of the ratio of the reagents is necessary to prevent the occurrence of a polymerisation process triggered by the exothermic character of the hydrolysis reaction [7].

The formation of secondary products, such as salts deriving from neutralisation, is a further drawback which imposes additional costs related to the disposal of the chemical waste produced. Accordingly, there is a strong interest in the development of new, more efficient amide production strategies capable of overcoming the limitations of the currently available procedures, above all on an industrial scale. In this area, some interesting results have been obtained using metallic or enzymatic catalysts [4][8]. However, these alternative approaches are still hampered by significant complications, due for example to the use of carbon monoxide [4], the need to use coordination complexes which are not commercially available as catalysts [8], or enzymes with low thermal stability and applicability restricted to a few substrates [8].

The document by Tao Wang et al., 2019 ("Preparation of benzamide by hydration of benzonitrile with palladium acetate and phenylarsonic acid as synergistic catalyst", Chinese Journal of Inorganic Chemistry, vol. 35, no.9) is also known. This document describes a process for the production of benzamide through the hydration of benzonitrile, using phenylarsonic acid as a co-catalyst.

Also known is document JP S5289619, which describes the preparation of a saturated nitrile by means of a catalyst containing palladium. In particular, the document describes the preparation of the catalyst by calcination at 450°C of a palladium(II) species, with silica and arsenic, under a hydrogen atmosphere. It is known to the person skilled in the art that, under these reducing conditions, palladium(II) is converted into its metallic form. Therefore, the active palladium species described in this document is a dispersion of metallic palladium supported by silica.

In the light of the above, it appears evident that there is a need to be able to have a process for the preparation of amides that is capable of overcoming the disadvantages of the known processes.

The solution offered by the present invention fits into this context; it aims to provide an advantageous process for the preparation of amides.

In particular, the present invention concerns a nitrile hydration process based on a palladium/arsenic-mediated catalytic cycle capable of advantageously converting said nitriles, both aliphatic and aromatic, into the respective amides.

The catalytic hydration process according to the present invention uses inexpensive, commercially available materials, such as palladium chloride and arsenic trioxide, which in a solution lead to the formation of the catalytically active heterobimetallic complex. Furthermore, the catalytic cycle is effective at a neutral pH and low temperatures (such as for example 60 °C) and in presence of atmospheric oxygen. Thanks to these exceptionally mild conditions, no by-products are generated through the process of the present invention. Furthermore, the unreacted nitrile can be completely recovered by means of classic procedures such as recrystallisation, distillation or column chromatography.

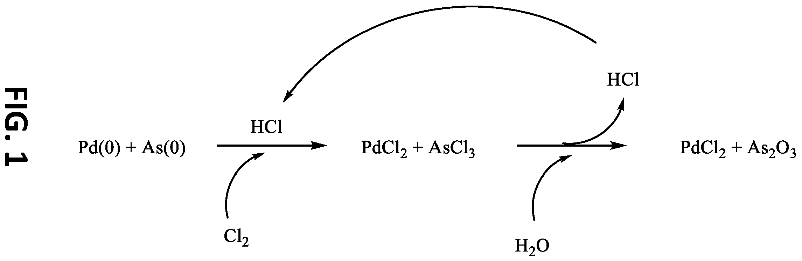

In the catalytic process developed, the substrate (nitrile) is mixed with an excess of water, with or without the presence of a suitable cosolvent, according to the solubility thereof in an aqueous environment. The mixture is heated to a temperature comprised in the range between 20 °C and 100 °C, and then a generator of arsenous acid (such as for example arsenic trioxide) and a generator of palladium(II) (such as for example $PdCl_2$) are added in a catalytic amount. The formation of the hydration product can be monitored by means of the most elementary analytic techniques (TLC) or using more complex methods (HPLC, GC-MS, NMR). The reaction reaches equilibrium in a period comprised between 2 and the 24 h, based on the substrate used. The recovery of the product can be achieved by means of column chromatography, crystallisation, precipitation, sublimation or distillation (depending on the nature of the synthesised product). Finally, the catalytic system, which during the reaction can be liable to deactivation due to the chemical reduction of the metals, can be regenerated by dissolution of the residual metal powder with HCl and $Cl_2$ and subsequent hydrolysis of $AsCl_3$ according to the scheme illustrated in figure 1.

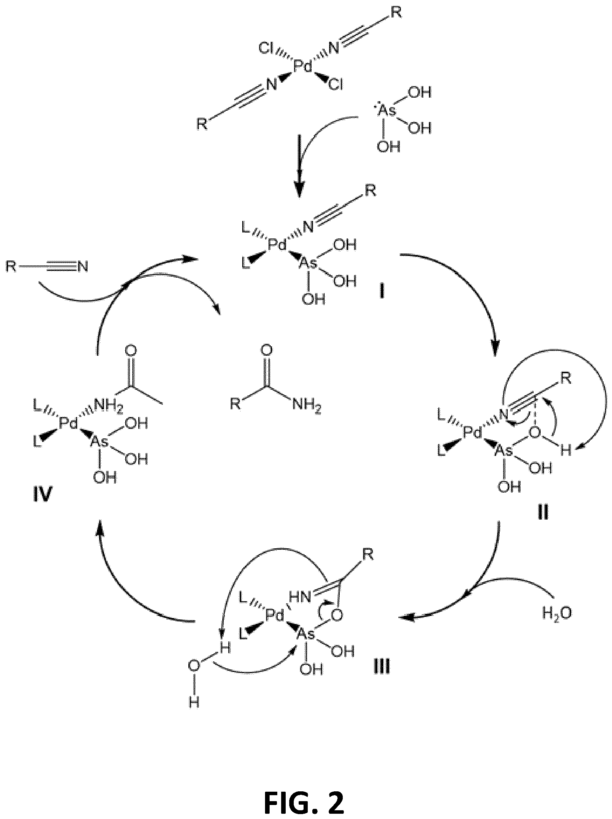

The hypothesised catalytic scheme of the process according to the present invention is illustrated in figure 2.

The hypothesised reaction mechanism finds support in the following observations:

- the reaction is not effective in the presence of solely the $Pd^{2+}$ generator (for example palladium chloride) or of solely the arsenous acid generator (for example arsenic trioxide). Hydration takes place only if both catalysts are present in the reaction environment;
- there exist examples regarding the direct $Pd-As(OH)_3$ bond [9], as well as the presence of Pt-As motifs [10]. More precisely, the coordination bonds have already been reported in the literature and described through a robust X-ray diffraction approach [9][10].
- the reaction is not effective vis-à-vis substrates endowed with coordinating groups that are stronger relative to the nitrile fraction. For the purposes of the conversion, the coordination of the nitrile nitrogen at the palladium centre is essential. It follows that the presence, on the substrate, of functions/groups characterised by greater affinity for the metallic centre than the nitrile group itself determines a substantial decrease in the effectiveness of the catalytic process of formation of the corresponding amide. In fact, in these cases there is a competition for coordination at the palladium centre. This behaviour has been verified with pyridine, pyrimidine or cationic substrates (pyridinium salts) with a coordinating iodide as a counterion. Therefore, all this suggests that the coordination of the nitrile group on the palladium atom is a necessary step for the start of the catalytic cycle;
- the reaction is strongly promoted by the presence of electron withdrawing groups (EWGs) conjugated to the nitrile portion, such as nitro or N-methylpyridinium groups. The latter aspect can be explained on the basis of step II (Fig. 2) of the proposed reaction mechanism, in which the nucleophilic attachment of oxygen on the nitrile carbon atom is favoured by a greater positive charge density induced by the EWG substituents;
- the intermediate obtained in step III (Fig. 2) can be isolated after substitution of the palladium with a less reactive isoelectronic metal centre [10]. In this case, it is possible to isolate an intermediate wholly similar to the one proposed in step III (Fig.2), which we propose as the catalytically active species.

The reactivity of the catalytic pair (e.g. $PdCl_2/As_2O_3$), used in a 1:1 molar ratio, remains constant up to a molar ratio of 1:3200 relative to the nitrile substrate. Once this ratio of the catalytic/substrate pair has been fixed, a reduction of one of the two components ($PdCl_2$; $As_2O_3$) results in a progressive reduction in yield. An increase in one of the two components, by contrast, does not modify the conversion yield.

According to the present invention, the acetonitrile hydration reaction was also conducted on a broader scale to assess the suitability of the process to a larger amount of substrate and estimate the turnover number (TON) of the arsenic-palladium catalytic complex. In particular, two experiments were carried out on a large scale. In the first reaction, the good yield (41% versus 48% of the smaller configuration) was confirmed, whilst through the second experiment the turnover number of the catalytic system was calculated as $1.86 \times 10^3$.

The process according to the present invention is advantageous compared to the known processes, as it is conducted under mild conditions, i.e. a neutral pH and low temperatures, and does not result in the production of by-products.

For example, compared to the process described in the document by Tao Wang et al., 2019, which is a process suitable solely for the hydration of benzonitrile, the process according to the invention is applicable to a broader spectrum of substrates. Furthermore, the process described in Tao Wang et al., 2019, uses phenylarsonic acid as a cocatalyst, which, under the reaction conditions described by Tao Wang et al., is not an arsenous acid generator. The presence of said arsenous acid is, by contrast, necessary in the chemical process described in the present invention. Furthermore, according to the present invention, it is possible to recover both the palladium and the arsenic from the exhausted catalyst in the form, respectively, of palladium chloride and arsenic trioxide, unlike what is reported in the document by Tao Wang et al., in which no mechanism for the recovery of the catalyst is proposed.

Furthermore, as mentioned above, the process according to the present invention has the advantage of being able to be conducted at much lower temperatures than the ones used in processes of the prior art, such as, for example, in the process described in JP S5289619, wherein the process works only at temperatures of between 200 and 700°C. The use of a much lower temperature is a large advantage in an industrial expansion, since a lower temperature means less energy consumption and less thermomechanical stress on the reaction apparatus. In this context, the temperature reduction thus represents an economically advantageous modification. Moreover, the process described in JP S5289619 uses a different process based, as mentioned above, on a dispersion of metallic palladium supported by silica.

It is therefore, a specific object of the present invention a process for preparing an amide by hydration of a nitrile, said process comprising or consisting of the following steps:

a) mixing the nitrile with water or with water and a suitable cosolvent in order to solubilise said nitrile;

b) bringing the solution obtained in step a) to a temperature comprised in the range from 20°C to 100°C;

c) adding a catalytic amount of a catalytic system capable of producing arsenous acid *in situ,* said catalytic system comprising or consisting of a first compound and a second compound, said first compound being an arsenous acid generator compound and said second compound being selected from a palladium(II) generator and a palladium (II) compound, said palladium (II) being able to be coordinated by the arsenic atom of said arsenous acid, and allowing to react until the formation of the amide.

According to the process of the present invention, it is preferable that the nitrile (and also the reaction environment) does not comprise other groups capable of coordinating palladium(II) more effectively than the nitrile group. More specifically, according to the process of the present invention, it is preferable that the nitrile does not bear other coordinating groups which compete with the nitrile nitrogen for the coordination of the palladium. For example, it is preferable that the nitrile does not comprise pyridine, pyrimidine or cationic substrates (such as for example pyridinium salts) with a coordinating iodide as a counterion.

According to the present invention, it is essential that arsenous acid is produced, thanks to said arsenous acid generator. Arsenous acid is characterised by an unshared (or lone) electron pair, which enables the establishment of coordinative bonds between the arsenic centre and other atoms that behave like Lewis acids. In particular, in the process of the present invention, the presence of said unshared electron pair of the arsenous acid generated enables the formation of a direct arsenic-palladium (II) coordinative bond.

According to one embodiment of the present invention, said arsenous acid generator can be a compound capable of acting as a generator of arsenous acid in an aqueous environment. In particular, said arsenous acid generator can preferably be a compound capable of directly generating arsenous acid in an aqueous environment, such as for example arsenic trioxide.

The person skilled in the art is capable of selecting a suitable arsenous acid generator through routine means and methods at his disposal. Furthermore, it is well known to the person skilled in the art that it is possible to add any further compounds, such as, for example, acids, reducing agents or oxidising agents, that may be necessary so that the selected arsenous acid generator gives rise to the arsenous acid necessary to catalyse the reaction.

According to some embodiments of the process according to the present invention, the nitrile has formula (I):

R-CN        (I)

wherein the R group is selected from an aliphatic, aromatic, benzyl, allyl, vinyl, or methacrylic group.

Preferably, said R group can be functionalised with one or more electron-withdrawing groups capable of rendering the nitrile carbon more electrophilic by conjugating thereto. Even more preferably, said one or more electron-withdrawing groups are in a vicinal position with respect to the nitrile group. For example, when the R group is an aromatic group, the electron-withdrawing group can be in an ortho or para position of the aromatic ring (which in turn bears the nitrile group directly bonded), whereas when R is an aliphatic group, the electron-withdrawing group can be in an alpha position with respect to the nitrile group. Said one or more electron-withdrawing groups can be selected from a nitro group, a halogen, such as chlorine, fluorine, bromine, iodine, or a phenyl. In the case of phenyl, the nitrile preferably consists of an aromatic nitrile with a benzene ring as a substituent in the alpha position of the main chain. Said functional group of the main aliphatic chain is capable of favouring the reaction according to the present invention, since the aromatic function is configured as an electron-withdrawing group.

The nitriles can be liquid and water-miscible nitriles, liquid hydrophobic nitriles, and solid nitriles.

As a suitable cosolvent for solubilising the nitrile, use can be made of DMF and solvents with characteristics similar to DMF, namely polar aprotic solvents with a low coordinating capacity towards the palladium centre, whereas it is preferable not to use non-polar or scarcely polar protic solvents such as alcohols and THF.

According to the process of the present invention, in general it is not necessary to adjust the pH. The pH that arises during the reaction is about 7, and this pH assures maximum tolerance towards the substrates.

According to the present invention, the temperature of step b) can be a temperature preferably comprised in the range of 20-60 °C, more preferably 30-60°C, even more preferably 40-50 °C.

According to the process of the present invention, the catalytic system is added in catalytic amounts, as known to the person skilled in the art, i.e. in an amount that is extremely low compared to the substrate. Preferably, a catalytic system:substrate ratio of up to 1:3200 can be used, i.e. each molecule of the $Pd^{2+}$ generator compound or of the palladium(II) compound, such as for example $PdCl_2$, and each molecule of the arsenous acid generator, such as for example $As_2O_3$ preferably requires 3200 molecules of the substrate, that is, of nitrile. Further increasing the substrate relative to the catalytic system could decrease the reaction yield.

According to the present invention, the arsenous acid generator can be selected from the group consisting of $As_2O_3$ and an arsenite, for example an arsenite with an alkali or alkaline earth metal. If an arsenite is used, the reaction environment must be acidified in order to generate arsenous acid.

According to the process of the present invention, the Pd(II) generator compound is a Pd compound capable of generating, in a solution, the presence of $Pd^{2+}$ complexes bearing mono- or polydentate ligands.

The person skilled in the art is capable of selecting a suitable Pd(II) generator through routine means and methods at his disposal. Furthermore, it is well known to the person skilled in the art that it is possible to add any further compounds, such as, for example, oxidising or reducing agents, that may be necessary so that the selected Pd(II) generator gives rise to the Pd(II) necessary to catalyse the reaction. The $Pd^{2+}$ that is generated must be stable, i.e. it must not be further reduced or oxidised, otherwise the reaction according to the present invention would not take place. Therefore, in the reaction environment use must not be made of strong reducing or oxidising agents capable of reducing or oxidising the $Pd^{2+}$ necessary for the reaction.

According to the present invention, the Pd(II) generator can be selected in the group consisting of a Pd(IV) compound, a Pd(0) compound, and Pd(0). For example, according to one embodiment of the invention, Pd(0) nanostructures can be used. Therefore, in the catalytic system according to the process of the present invention, the palladium(II) component is obtained by using a palladium(II) compound or a Pd(II) generating compound such as a Pd(IV) or Pd(0) compound, the latter being subjected respectively to a reduction or oxidation, with suitable reducing or oxidising agents, in order to obtain catalytically active $Pd^{2+}$ complexes.

The palladium(II) compound can be selected from the group consisting of a salt of Pd+2 with the formula $PdX_2$, wherein X is selected in the group consisting of Cl, Br, I, and NOs; $Pd(OTf)_2$(dppp); $Pd(OTf)_2$(dppe); palladium acetate; or $Pd(dppf)Cl_2$, wherein OTf stands for trifluoromethanesulfonic group, dppp stands for 1,3-bis(diphenylphosphino)propane group, dppe stands for 1,2-bis(diphenylphosphino)ethane group, and dppf stands for 1,1-bis(diphenylphosphino)ferrocene.

According to the present invention, the first compound and the second compound of the catalytic system can be added in a 1:1 ratio.

It remains understood that the reaction proceeds also with an excess of the arsenous acid generator relative to the $Pd^{2+}$ generator (and vice versa). However, in such a case a substantial waste of one or the other species would occur.

According to one embodiment of the process of the present invention, said process can further comprise the following step:

d) isolating the amide produced in step c) from the reaction environment.

Step d) can be carried out using a technique selected in the group consisting of column chromatography, crystallisation, precipitation, sublimation, and distillation.

According to a further embodiment of the process of the present invention, said process can further comprise the following step:

e) regenerating the catalytic system. In particular, said step e) preferably provides for recovering the arsenic as $As_2O_3$. Furthermore, said step e) preferably provides for recovering the palladium as palladium chloride.

Therefore, the process of the present invention can be a cyclic process. In such a case, after step e) it again follows step a).

The invention will be described below by way of non-limiting illustration, with particular reference to the illustrative examples and the appended figures, wherein:

- figure 1 shows a regeneration scheme of the catalytic system;
- figure 2 shows the hypothesised catalytic scheme of the process according to the present invention;

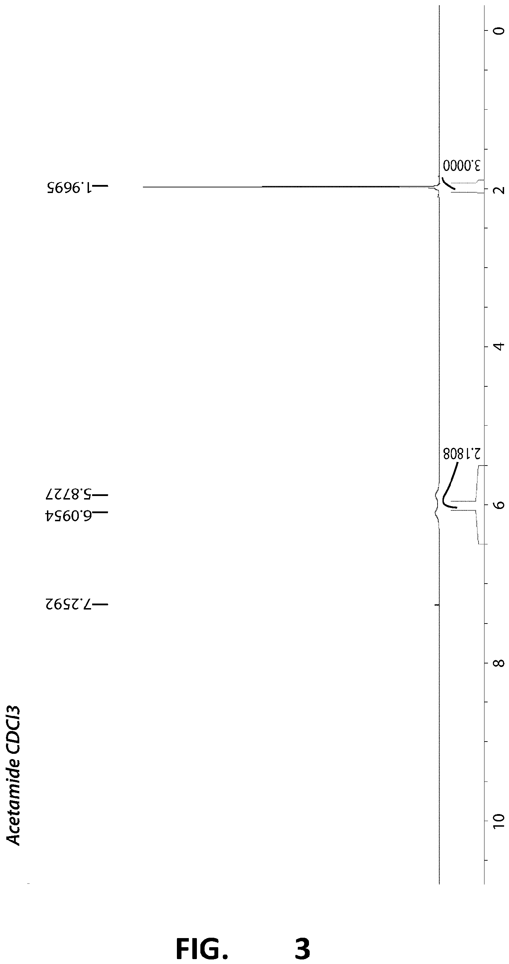

- figure 3 shows the $^1$HNMR spectrum of the acetamide produced according to the present invention; (500 MHz; $CDCl_3$) δ: 6.09 (1H, b); 5.87 (1H, b); 1.97 (3H, s);

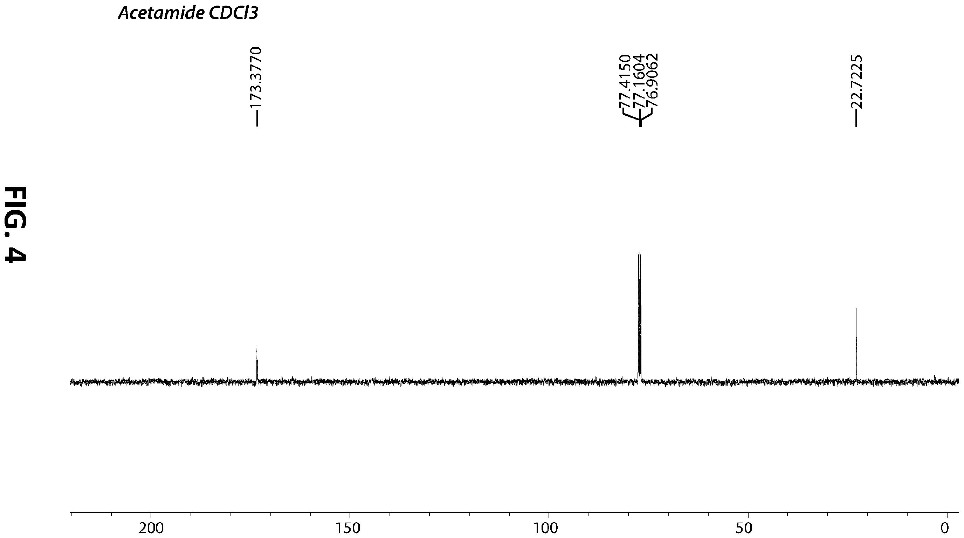

- figure 4 shows the $^{13}$CNMR spectrum of the acetamide produced according to the present invention; (125 MHz;

CDCl$_3$) δ: 173.4; 22.7;

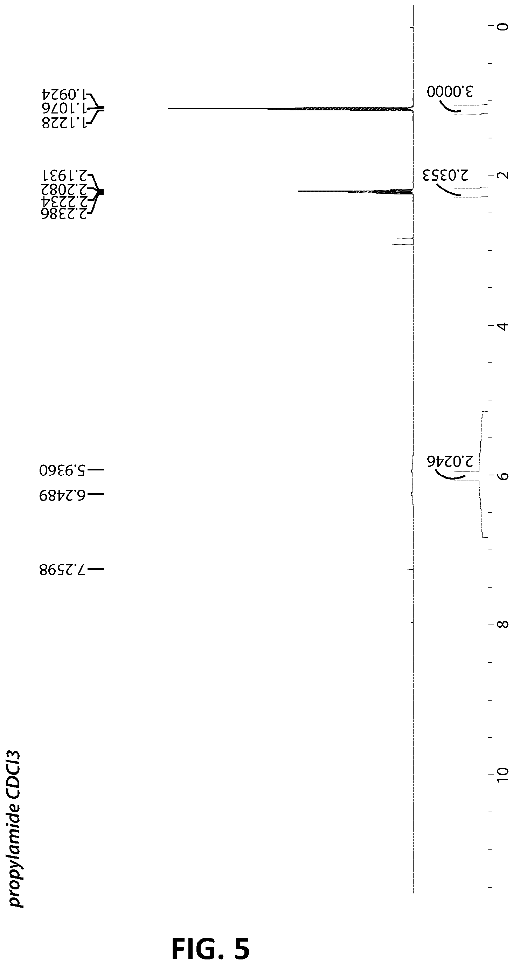

- figure 5 shows the $^1$HNMR spectrum of the propanamide produced according to the present invention: (500 MHz; CDCl$_3$) δ: 6.25 (1H, b); 5.94 (1H, b); 2.21 (2H, q, *J*= 7.59 Hz); 1.11 (3H, t, *J*= 7.59 Hz);

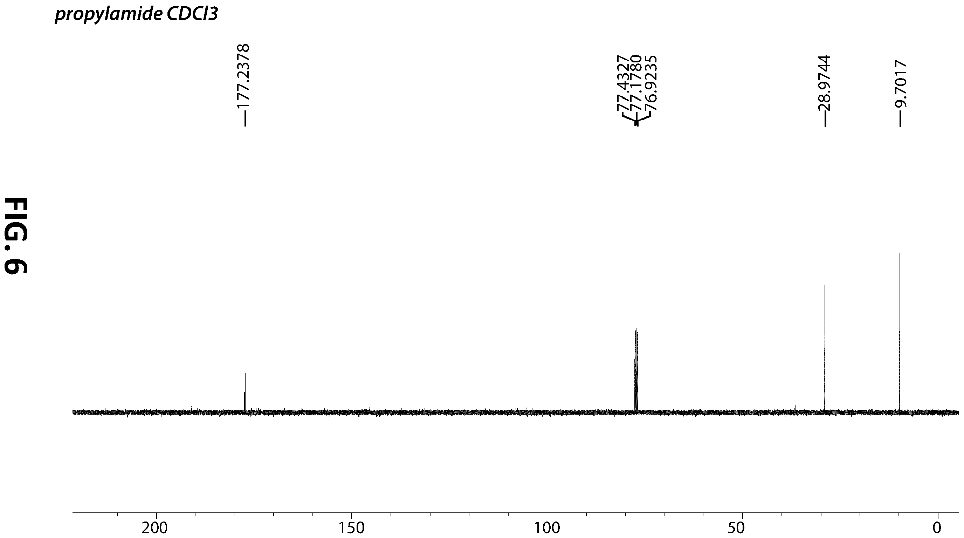

- figure 6 shows the $^{13}$CNMR spectrum of the propanamide produced according to the present invention: (125 MHz; CDCl$_3$) δ: 177.2; 28.9; 9.7;

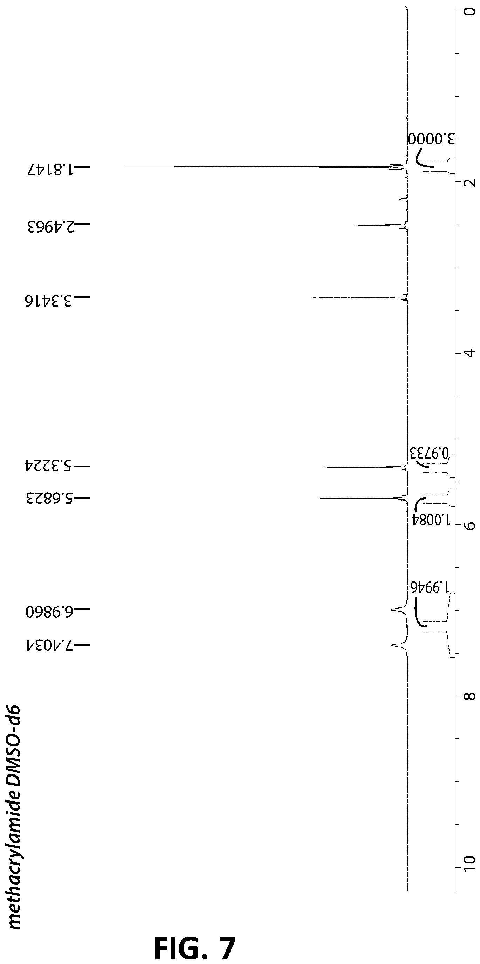

- figure 7 shows the $^1$HNMR spectrum of the methacrylamide produced according to the present invention: (500 MHz; DMSO-d$_6$) δ: 7.40 (1H, b); 6.99 (1H, b); 5.68 (1H, s); 5.32 (1H, s); 1.81 (3H, s);

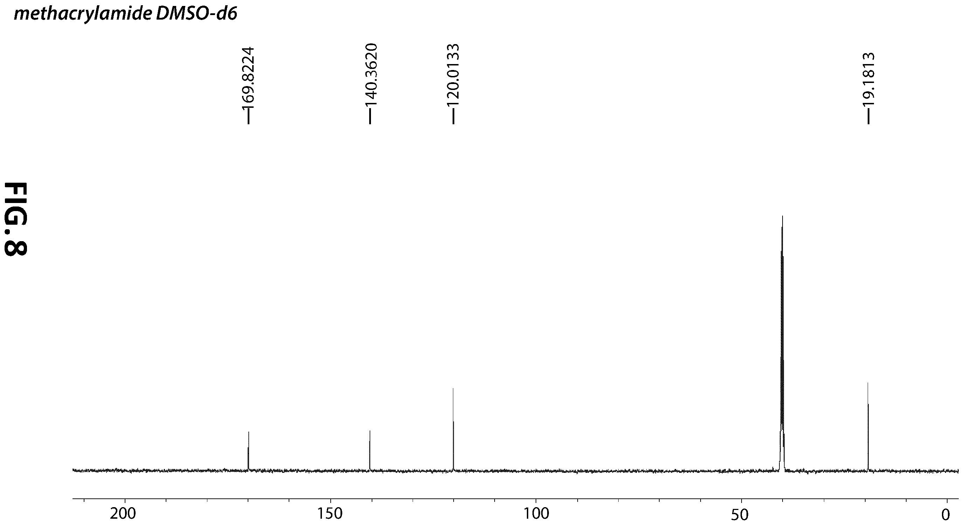

- figure 8 shows the $^{13}$CNMR spectrum of the methacrylamide produced according to the present invention: (125 MHz; DMSO-d$_6$) δ: 169.8; 140.4; 120.0; 19.2;

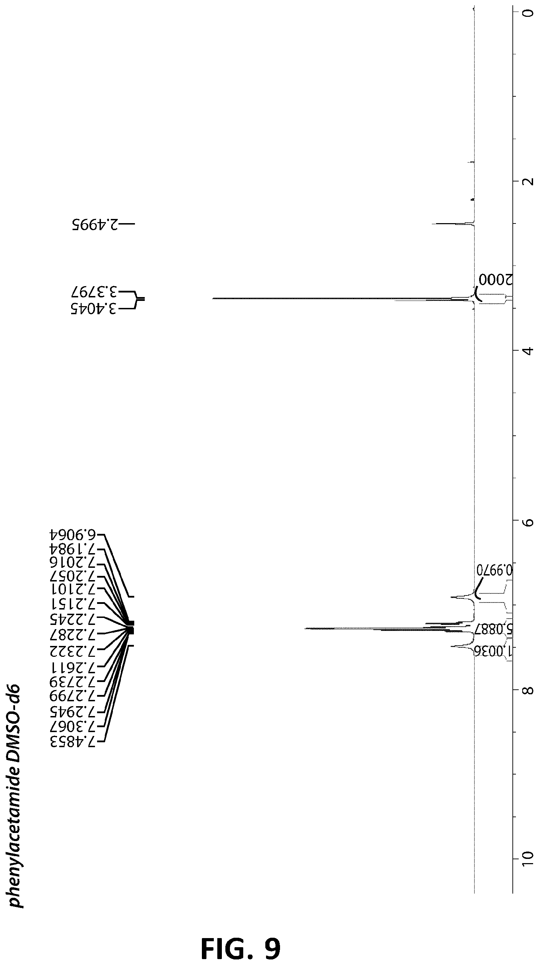

- figure 9 shows the $^1$HNMR spectrum of the phenylacetamide produced according to the present invention: (500 MHz; DMSO-d$_6$) δ: 7.48 (1H, b); 7.27 (4H, m); 7.21 (1H, m); 6.91 (1H, b); 3.40 (2H, s);

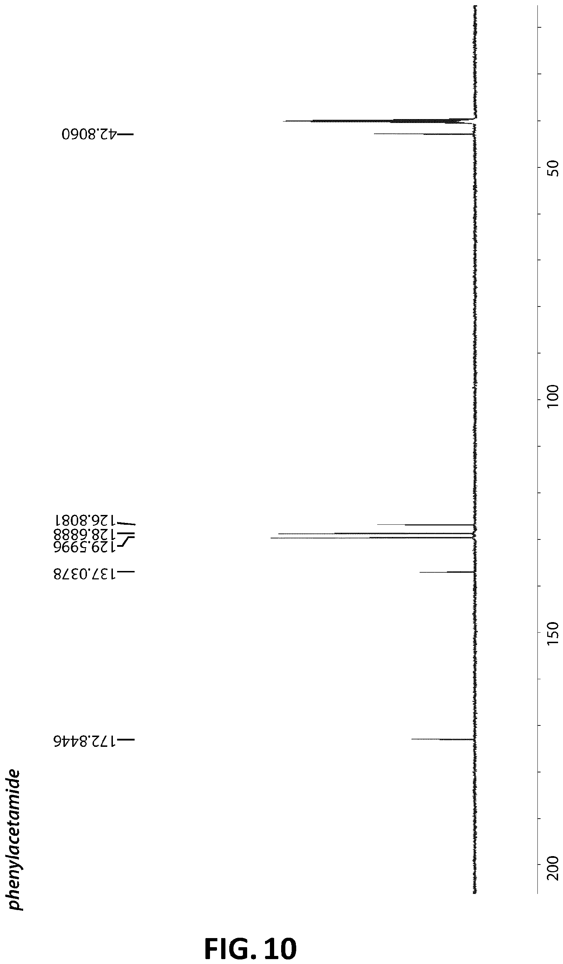

- figure 10 shows the $^{13}$CNMR spectrum of the phenylacetamide produced according to the present invention: (125 MHz; DMSO-d$_6$) δ: 172.8; 137.0; 129.6; 128.7; 126.8; 42.8;

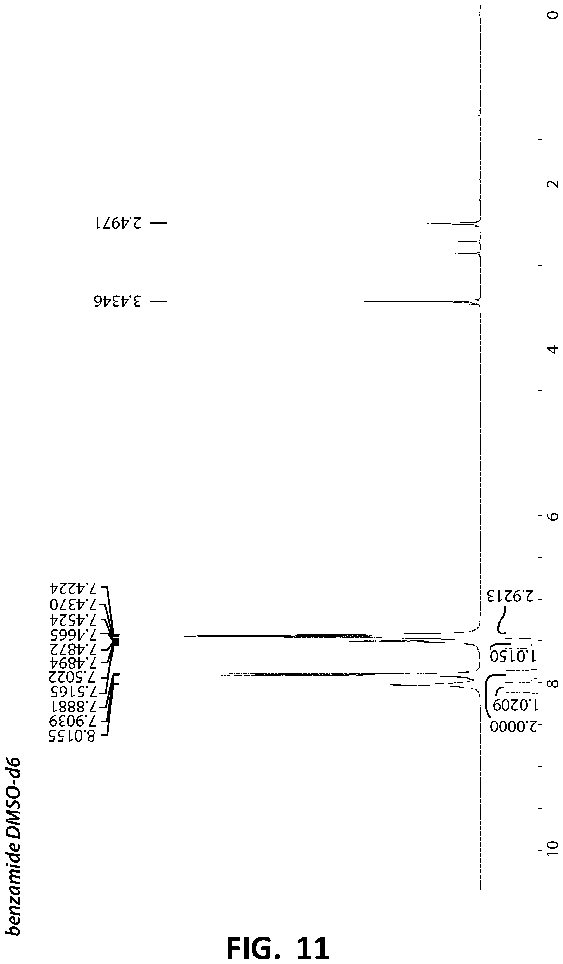

- figure 11 shows the $^1$HNMR spectrum of the benzamide produced according to the present invention: (500 MHz; DMSO-d$_6$) δ: 8.01 (1H, b); 7.89 (2H, d, *J*= 7.90); 7.50 (1H, t, *J*= 7.32 Hz); 7.44 (3H, m);

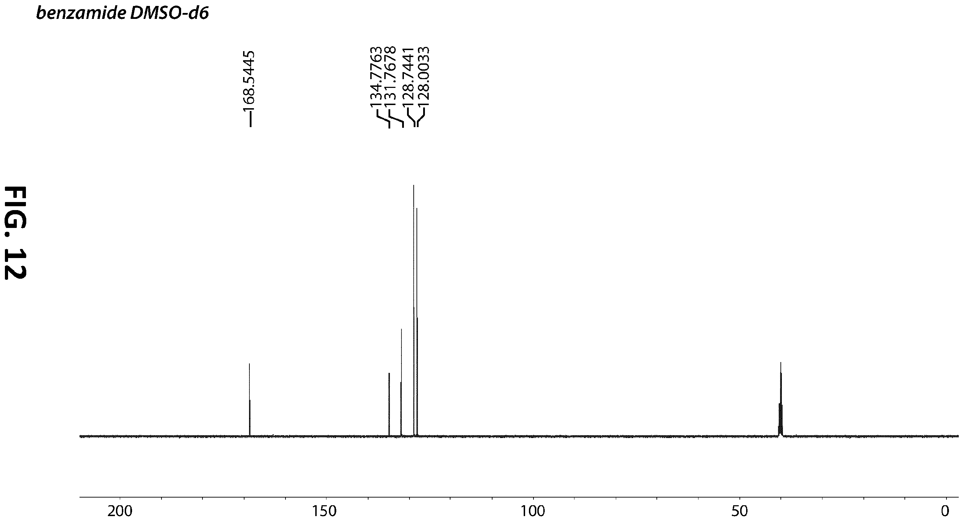

- figure 12 shows the $^{13}$CNMR spectrum of the benzamide produced according to the present invention: (125 MHz; DMSO-d$_6$) δ: 168.5; 134.8; 131.8; 128.7; 128.0;

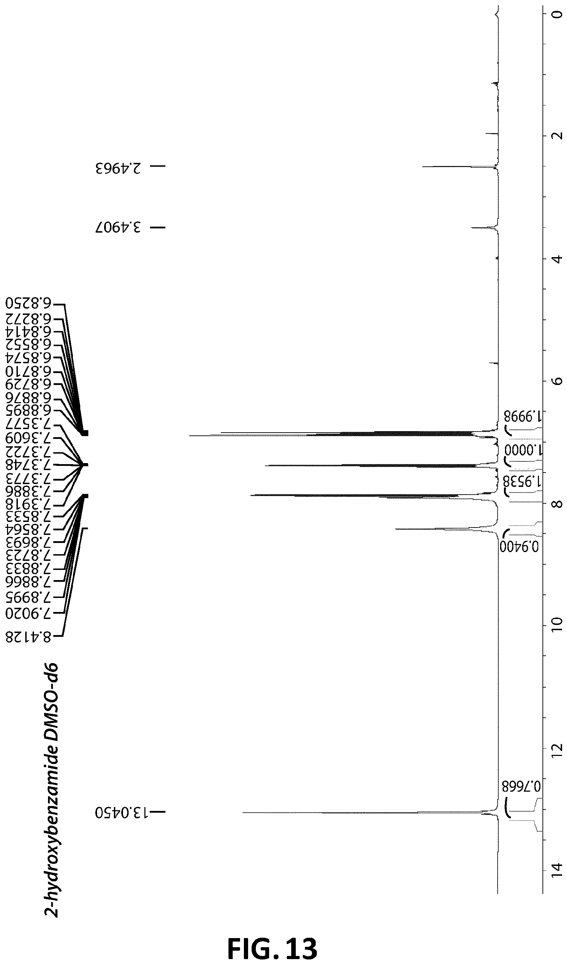

- figure 13 shows the $^1$HNMR spectrum of the 2-hydroxybenzamide produced according to the present invention: (500 MHz; DMSO-d$_6$) δ: 13.04 (1H, s); 8.41 (1H, b); 7.90 (1H, b); 7.86 (1H, dd, *J*$_3$= 7.96 Hz, *J*$_4$= 1.53 Hz); 7.37 (1H, m); 6.88 (1H, dd, *J*$_3$= 8.29 Hz, *J*$_4$= 0.94 Hz); 6.84 (1H, m);

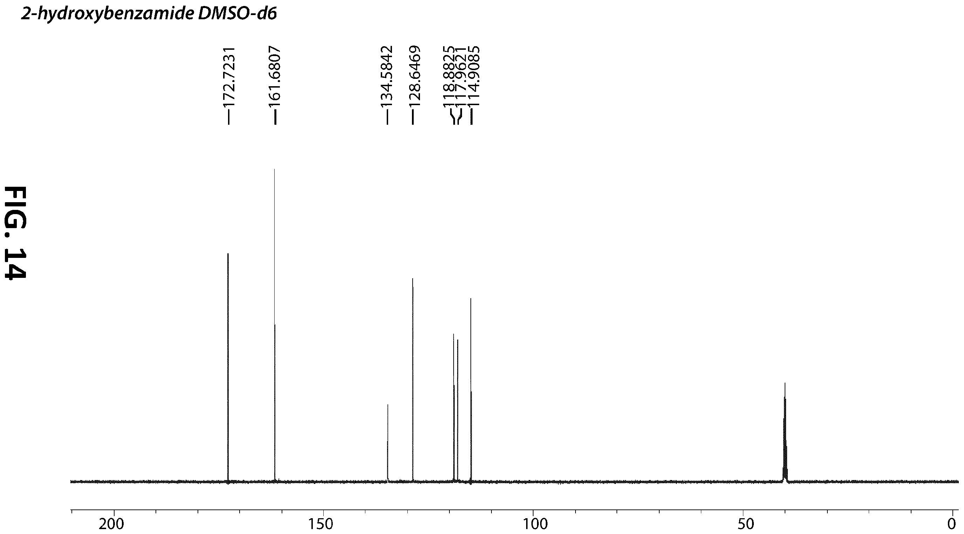

- figure 14 shows the $^{13}$CNMR spectrum of the 2-hydroxybenzamide produced according to the present invention: (125 MHz; DMSO-d$_6$) δ: 172.7; 161.7; 134.6; 128.6; 118.9; 117.9; 114.9;

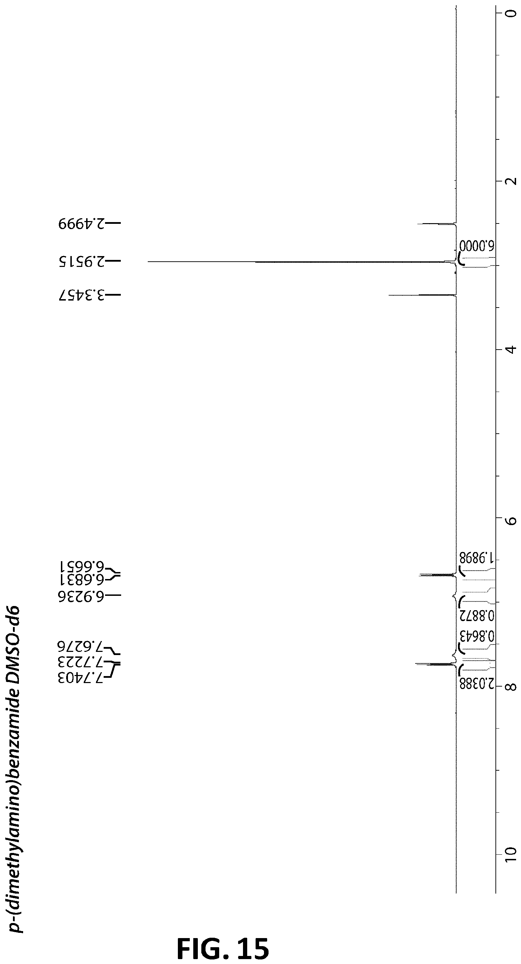

- figure 15 shows the $^1$HNMR spectrum of the 4-(dimethylamino)benzamide produced according to the present invention: (500 MHz; DMSO-d$_6$) δ: 7.73 (2H, d, *J*= 8.99 Hz); 7.63 (1H, b); 6.92 (1H, b); 6.67 (2H, d, *J*= 8.99 Hz); 2.95 (6H, s);

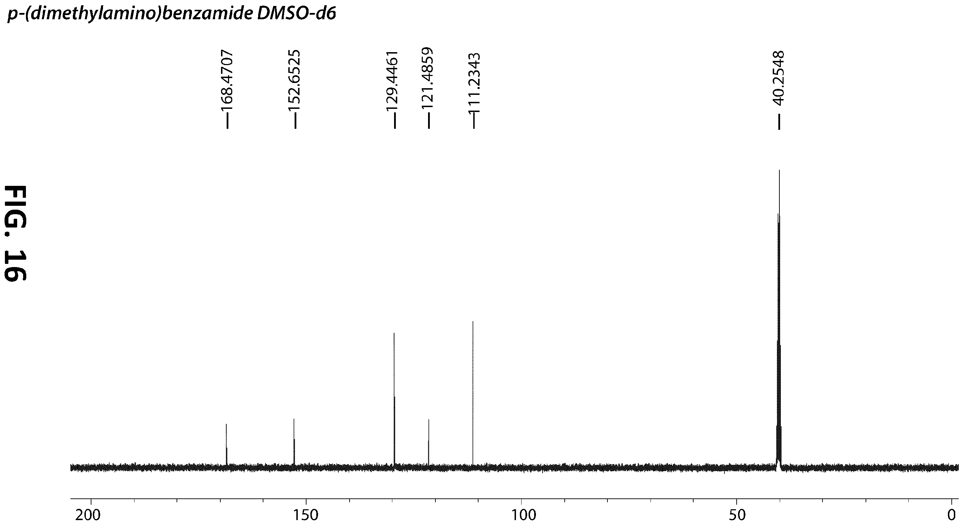

- figure 16 shows the $^{13}$CNMR spectrum of the 4-(dimethylamino)benzamide produced according to the present invention: (125 MHz; DMSO-d$_6$) δ: 168.5; 152.6; 129.4; 121.5; 111.2;

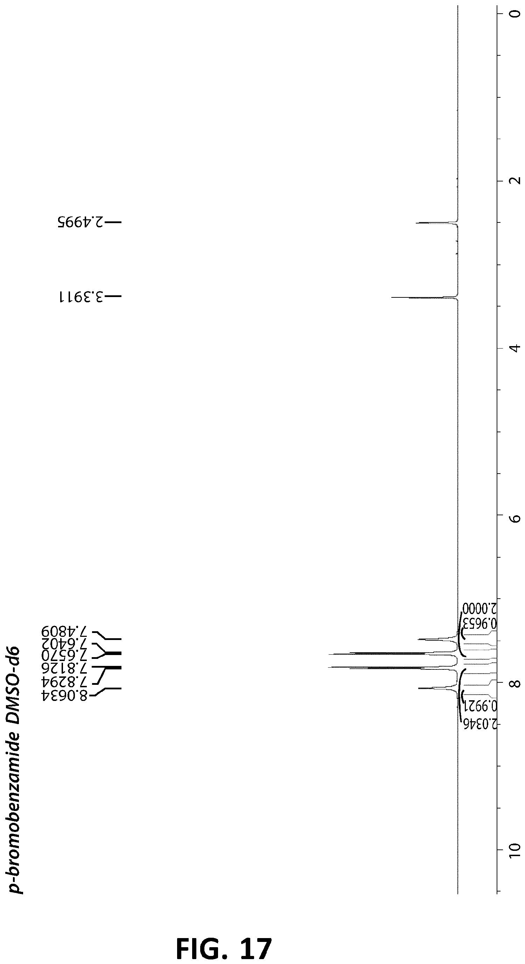

- figure 17 shows the $^1$HNMR spectrum of the 4-bromobenzamide produced according to the present invention: (500 MHz; DMSO-d$_6$) δ: 8.06 (1H, b); 7.82 (2H, d, *J*= 8.40 Hz); 6.65 (2H, d, *J*= 8.40 Hz); 7.48 (1H, b);

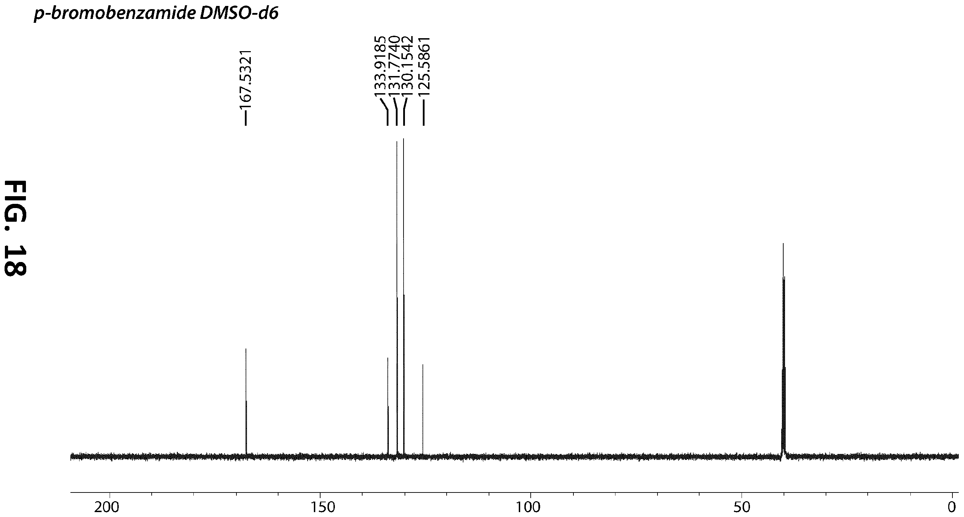

- figure 18 shows the $^{13}$CNMR spectrum of the 4-bromobenzamide produced according to the present invention: (125 MHz; DMSO-d$_6$) δ: 167.5; 133.9; 131.8; 130.2; 125.6;

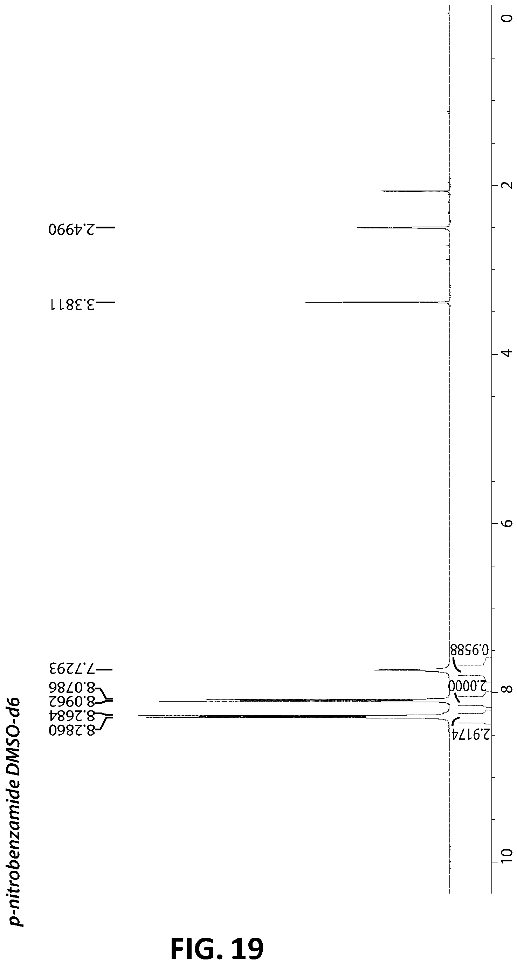

- figure 19 shows the $^1$HNMR spectrum of the 4-nitrobenzamide produced according to the present invention; (500 MHz; DMSO-d$_6$) δ: 8.28 (3H, m); 8.09 (2H, d, *J*= 8.80 Hz);

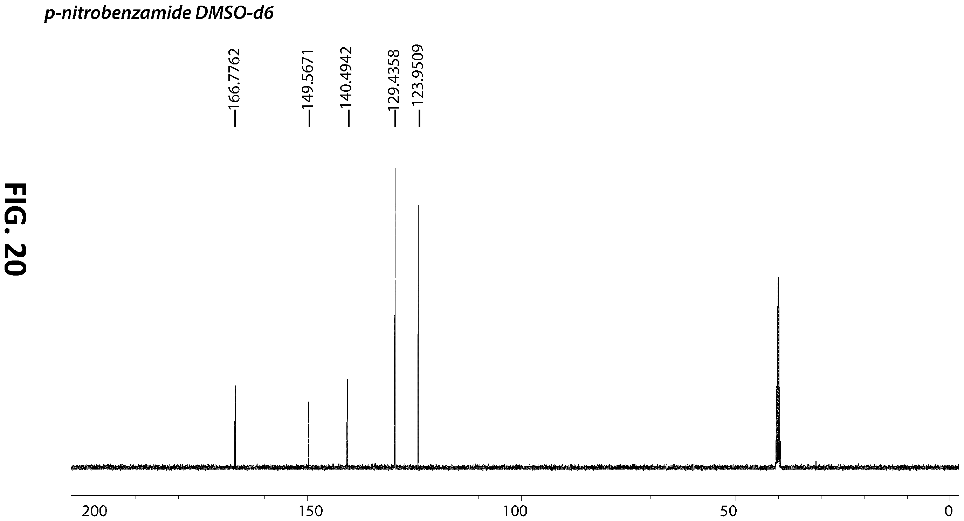

- figure 20 shows the $^{13}$CNMR spectrum of the 4-nitrobenzamide produced according to the present invention: (125 MHz; DMSO-d$_6$) δ: 166.8; 149.6; 140.5; 129.4; 123.9;

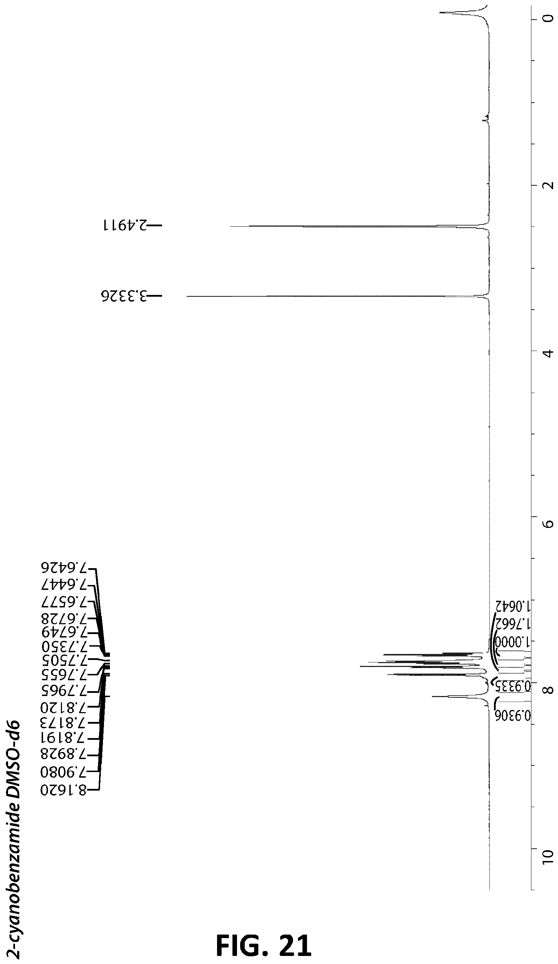

- figure 21 shows the $^1$HNMR spectrum of the 2-cyanobenzamide produced according to the present invention; (500 MHz; DMSO-d$_6$) δ: 8.16 (1H, b); 7.90 (1H, d, *J*= 7.63 Hz); 7.80 (1H, m); 7.75 (2H, m); 7.66 (1H, m);

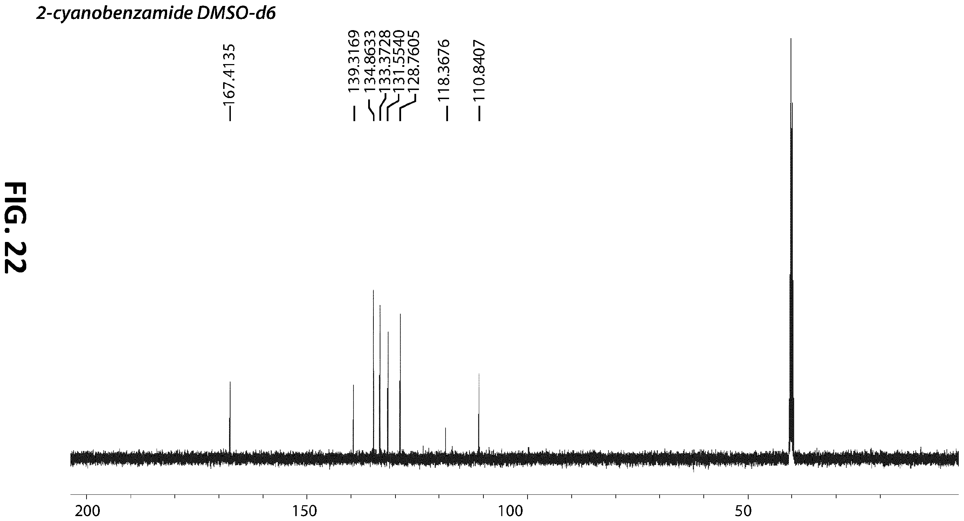

- figure 22 shows the $^{13}$CNMR spectrum of the 2-cyanobenzamide produced according to the present invention: (125 MHz; DMSO-d$_6$) δ: 167.4; 139.3; 134.9; 133.4; 131.6; 128.8; 118.4; 110.8;

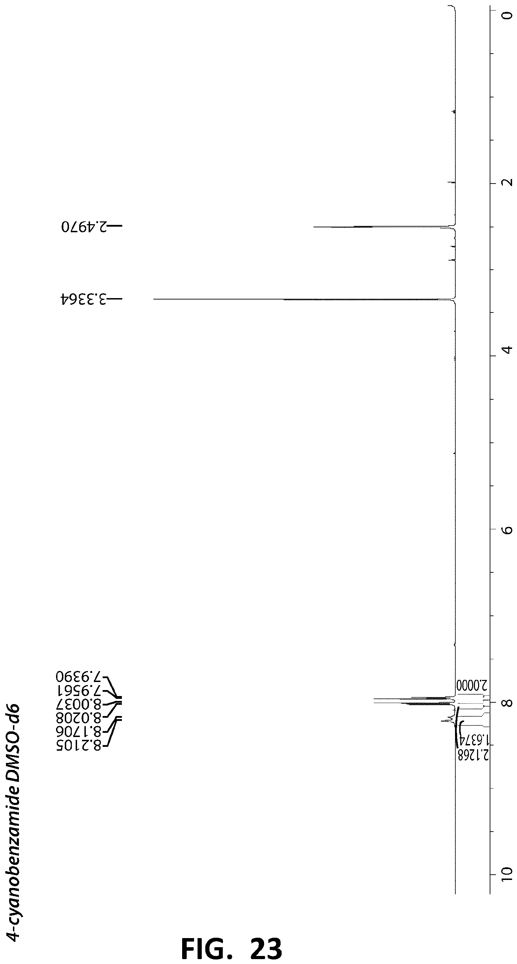

- figure 23 shows the $^1$HNMR spectrum of the 4-cyanobenzamide produced according to the present invention: (500 MHz; DMSO-d$_6$) δ: 8.21 (1H, b); 8.17 (1H, b); 8.01 (2H, d, *J*= 8.56 Hz); 7.95 (2H, d, *J*= 8.56 Hz);

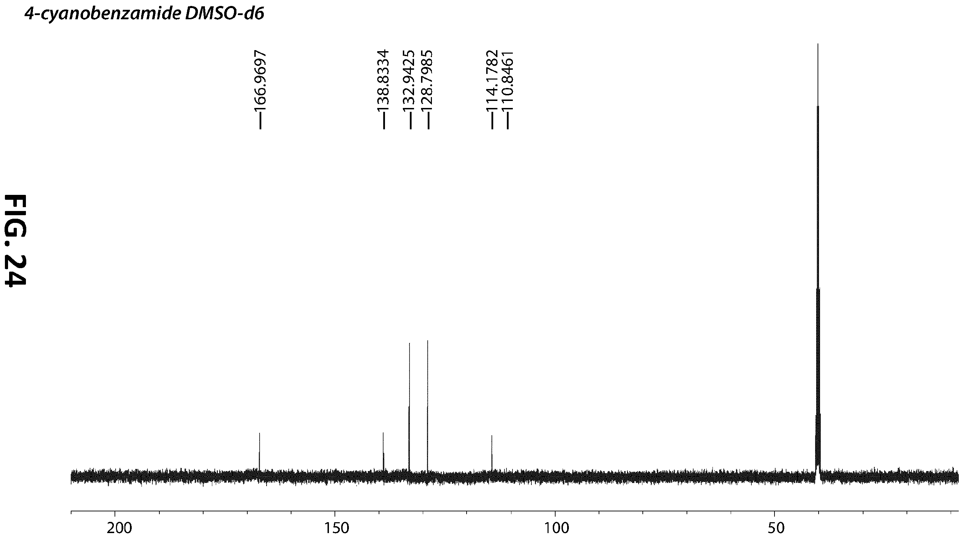

- figure 24 shows the $^{13}$CNMR spectrum of the 4-cyanobenzamide produced according to the present invention: (125 MHz; DMSO-d$_6$) δ: 166.9; 138.8; 132.9; 128.8; 114.2; 110.8; 118.4; 110.8;

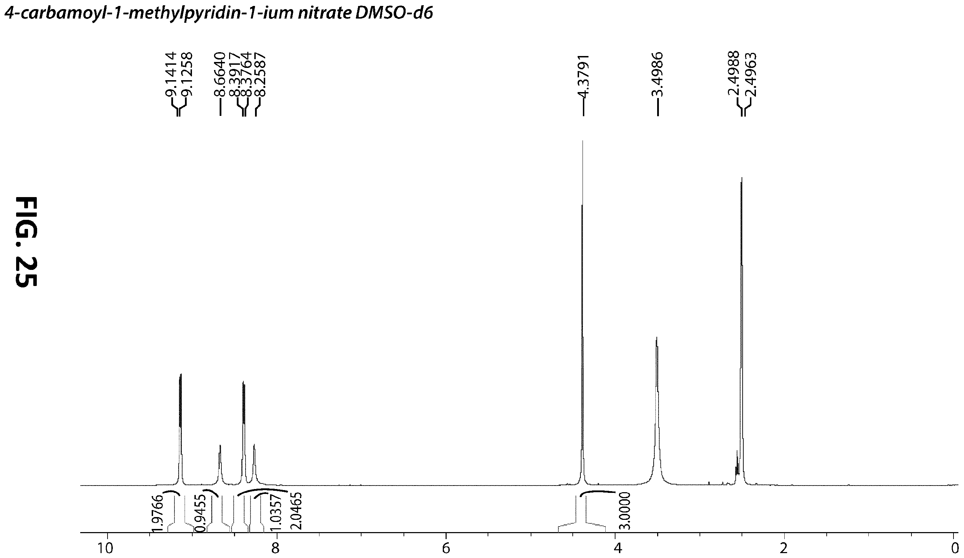

- figure 25 shows the $^1$HNMR spectrum of the 4-carbamoyl-1-methylpyridin-1-ium nitrate produced according to the present invention: (500 MHz; DMSO-d$_6$) δ: 9.13 (2H, d, *J*= 6.26 Hz); 8.66 (1H, b); 8.38 (2H, d, *J*= 6.12 Hz); 8.26 (1H, b);

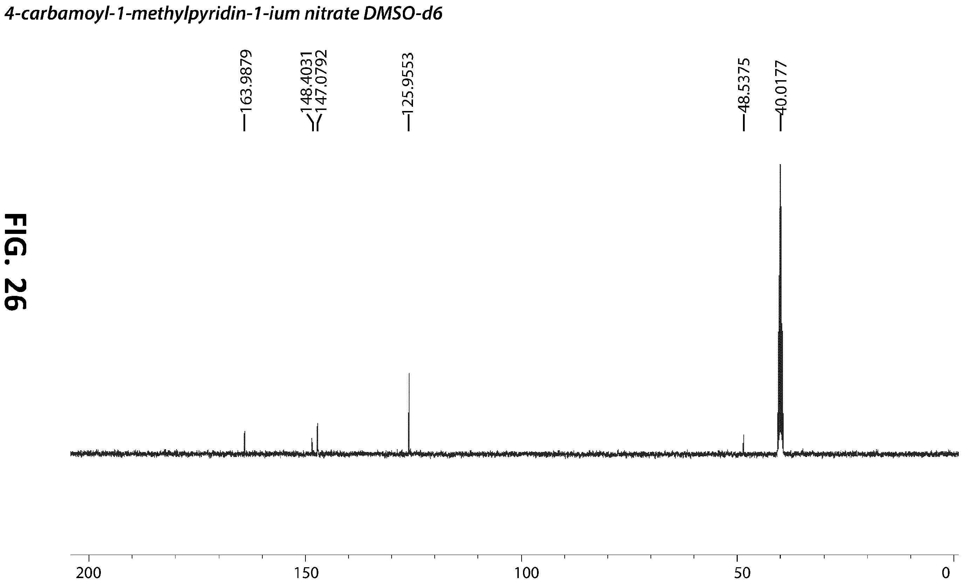

- figure 26 shows the $^{13}$CNMR spectrum of the 4-carbamoyl-1-methylpyridin-1-ium nitrate produced according to the present invention: (125 MHz; DMSO-d$_6$) δ: 163.9; 148.4; 147.1; 125.9; 48.5.

**EXAMPLE 1:** *process for preparing amides from nitriles according to the present invention*

The hydration process was carried out on sixteen different substrates.
Four different configurations were explored, namely A, B, C and D. These four procedures were used for liquid and water-miscible nitriles (A), liquid hydrophobic nitriles (B) and solid nitriles (C and D).

*Experimental procedure*

The reaction takes place as follows: a catalytic amount of the Pd$^{2+}$ generator (e.g. PdCl$_2$) is reacted with the selected nitrile to obtain the square planar complex bis(nitrile)dichloropalladium(II). Subsequently, an excess of water is added together with the arsenous acid generator (e.g. $As_2O_3$), in a 1:1 molar ratio relative to the amount of $Pd^{2+}$ initially generated. Under these conditions, $As_2O_3$ reacts with the water to form unstable arsenous acid. The latter, thanks to the availability of the lone electron pair, acts as a ligand towards the palladium atom. Therefore, it is presumed that the formation of a catalytic species takes place, wherein the palladium atom is capable of holding a molecule of nitrile in its coordination neighbourhood, and thus spatially near the molecule of arsenous acid (Figure 2, step I). As illustrated in the figure, one can assume that the hydroxyl groups bound to the arsenic atom can act as nucleophiles towards the nitrile carbon atom coordinated with the palladium (step II). Then, the reaction proceeds with the intervention of a water molecule (as illustrated in step III), causing the opening of the five-membered ring and the regeneration of the portion of arsenous acid that still remains coordinated at the Pd(II) centre (as illustrated at point IV). The coordination of a new nitrile molecule to the Pd(II) atom and the release of the amide formed complete the catalytic cycle.

*Results*

The study was conducted on a small scale (0.6 g - 1 g) on the substrates listed in table 1.

Table 1

| Substrate | Experimental setup | % amide yield |
|---|---|---|
| Acetonitrile | A | 48 |
| Propionitrile | B | 61 |
| Methacrylonitrile | B | 10 |
| Phenylacetonitrile | B | 56 |
| Benzonitrile | B | 54 |
| 2-hydroxybenzonitrile | C | 43 |
| 4-(Dimethylamino)benzonitrile | C | 34 |
| 4-Bromobenzonitrile | C | 70 |
| 4-Nitrobenzonitrile | C | 94 |
| Benzene-1,2-dicarbonitrile | C | 14 |
| Benzene-1,4-dicarbonitrile | D | 31 |
| 3-Pyridinecarbonitrile | C | 0 |
| 4-Pyridinecarbonitrile | C | 0 |
| 2-Pyrimidinecarbonitrile | C | 0 |
| 4-Cyano-1-methylpyridin-1-ium iodide | C | 0 |
| 4-Cyano-1-methylpyridin-1-ium nitrate | C | 100 |

Subsequently, with a view to a scale-up, tests were carried out on larger amounts of a selected substrate, using 130 g of acetonitrile. In this case as well, conversion percentages were obtained which were wholly comparable to those relating to the small scale (41% versus 48%), showing, at least on this larger scale, excellent maintenance of the activity of the catalytic pair.

The experimental details of the different reaction setups are given below and differ in the amount of dimethylformamide (DMF) used as a cosolvent:

*Setup A*

1 mL of acetonitrile and 10 mg of palladium chloride were added into a 50 mL round-bottom flask. The suspension was stirred and heated to 60 °C with an oil bath. After 30 minutes, 1 mL of demineralised water and 15 mg of arsenic trioxide were added. The suspension was stirred at 60 °C overnight; then the liquid phase in the flask was evaporated and the crude product was purified by classic flash column chromatography, eluting with 100% EtOAc.

*Setup B*

1 mL of the selected nitrile and 10 mg of palladium chloride were added into a 50 mL round-bottom flask. The suspension was stirred and heated to 60 °C with an oil bath. After 30 minutes, 1 mL of demineralised water, 0.5 mL of DMF and 15 mg of arsenic trioxide were added. The suspension was stirred at 60 °C overnight; then the liquid phase in the flask was evaporated and the crude products were purified by classic flash column chromatography (100% EtOAc for propionamide and methacrylamide; gradient of $CHCl_3$/EtOAc for phenylacetamide and benzamide).

*Setup C*

1 mL of the selected nitrile, 2 mL of DMF and 10 mg of palladium chloride were added into a 50 mL round-bottom flask. The suspension was stirred and heated to 60 °C with an oil bath. After 30 minutes, 0.5 mL of demineralised water and 15 mg of arsenic trioxide were added. The suspension was stirred at 60 °C overnight; then the liquid phase in the flask was evaporated and the crude products were purified by classic flash column chromatography (gradient of $CHCl_3$/EtOAc for 2-cyanobenzamide, 4-(dimethylamino)benzamide, 4-bromobenzamide; gradient of CHCIs/acetone for 4-nitrobenzamide; $CH_2Cl_2$/EtOAc 9:1 for 2-hydroxybenzamide) or simple filtering over a celite septum after dissolution in methanol (4-cyano-1-methylpyridin-1-ium nitrate).

*Setup D*

1 mL of the selected nitrile, 4 mL of DMF and 10 mg of palladium chloride were added into a 50 mL round-bottom flask. The suspension was stirred and heated to 60 °C with an oil bath. After 30 minutes, 1 mL of demineralised water and 15 mg of arsenic trioxide were added. The suspension was stirred at 60 °C overnight; then the liquid phase in the flask was evaporated and the crude product was purified by classic flash column chromatography with a gradient of $CHCl_3$/EtOAc.

*Large-scale setup 1*

170 mL of acetonitrile (132.6 g; 3.21 mol), 178 mg of palladium chloride (1 mmol), 198 mg of arsenic trioxide (1 mmol) and 60 mL of demineralised water were added into a 1 L round-bottom flask. The suspension was refluxed overnight at 110 °C in an oil bath. The suspension was then allowed to cool and subsequently filtered to remove the formed metal particles. The resulting solution was reduced in volume by means of a rotary evaporator and then vacuum dried until the formed acetamide was completely dried. With this setup, 76 g of acetamide were recovered; yield=41% (in good accordance with the one calculated in setup A).

*Large-scale setup 2*

This test was performed with the same methods as above, but with a reduction in the catalytic materials to 90 mg of palladium chloride (0.5 mmol) and 95 mg of arsenic trioxide (0.5 mmol). With this setup we observed a reduction in the yield to 30%, which allowed us to calculate the catalyst turnover number. TON: 1864.

The acetamide obtained in both large-scale setups was purified only by filtration, as these experiments served solely to confirm the robustness of the method and for TON calculation.

*References*

[1] J. E. Reimann and R. U. Byerrum in The Chemistry of Amides (Edited by J. Zabicky), John Wiley & Sons Ltd., London, 1970, Chapter 11, pp. 601-684.

[2] C. the Berre, P. Serp, P. Kalck, G. P. Torrence in Ullmann's Encyclopedia of Industrial Chemistry, Acetic Acid, Vol. 1, Wiley-VCH, Weinheim, 2014, pp. 231.

[3] a) S. Khalkar, D. Bhowmick, A. Pratap, J Oleo Sci. 2013, 62(11), 901-904. b) H. Low and J. E. Lauck in Amide Lubricants 1967, United States Patent Office, patent number 3,312,620.

[4] F. Messa, S. Perrone, M. Capua, F. Tolomeo, L. Troisi, V. Capriati, A. Salomone, Chem. Commun. 2018, 54, 8100-8103.

[5] C. A. G. N. Montalbetti, V. Falque, Tetrahedron 2005, 61, 10827-10852.

[6] a) J. N. Hahladakis, C. A. Velis, R. Weber, E. Iacovidou, P. Purnel, J. Haz. Mat. 2018, 344, 179-199. b) T. Ojeda in Polymer Science (Edited by F. Yilmaz), IntechOpen, 2013, Chapter 1.

[7] V. Y. Kukushin, A. J. L. Pombeiro, Inorg. Chim. Acta 2005, 358, 1-21.

[8] a) Z. Cheng, Y. Xia, Z. Zhou, Front. Bioeng. Biotechnol. 2020, 8, 352. b) A. R. Alcántara, P. Domínguez de Maria, J. A. Littlechild, M. Schürmann, R. A. Sheldon, R. Wohlgemuth, ChemSusChem, 2022, e202102709. c) M. C. Bryan, P. J. Dunn, D. Entwistle, F. Gallou, S. G. Koenig, J. D. Hayler, M. R. Hickey, S. Hughes, M. E. Kopach, G. Moine, P. Richardson, F. Roschangar, A. Steven, F. J. Weiberth, Green Chem., 2018, 20, 5082-5103.d) R. García-Álvarez, J. Francos, E. Tomás-Mendivil, P. Crochet, V. Cadierno, J. Organomet. Chem. 2014, 771, 93-104. e) E. Tomás-Mendivil, R. García-Álvarez, C. Vidal, P. Crochet, V. Cadierno, ACS Catal. 2014, 4, 1901-1910.

[9] M. N. Sokolov, A. V. Virovets, D. N. Dybtsev, E. V. Chubarova, V. P. Fedin, D. Fenske, Inorg. Chem. 2001, 40, 4816-1817.

[10] D. U. Miodragovic', J. A. Quentzel, J. W. Kurutz, C. L. Stern, R. W. Ahn, I. Kandela, A. Mazar, T. V. O'Halloran, Angew. Chem. Int. Ed. 2013, 52, 1-5.

**Claims**

1. A process for preparing an amide by hydration of a nitrile, said process comprising or consisting of the following steps:

    a) mixing the nitrile with water or with water and a cosolvent in order to solubilise said nitrile;
    b) bringing the solution obtained in step a) to a temperature comprised in the range from 20°C to 100°C;
    c) adding a catalytic system capable of producing arsenous acid *in situ,* said catalytic system comprising or consisting of a first compound and a second compound, said first compound being an arsenous acid generator compound and said second compound being selected from a palladium(II) generator and a palladium (II) compound, and allowing to react until the formation of the amide.

2. The process according to claim 1, wherein the nitrile has formula (I):

    R-CN            (I)

    wherein the R group is selected from an aliphatic, aromatic, benzyl, allyl, vinyl, or methacrylic group.

3. The process according to claim 2, wherein said R group is functionalised with one or more electron-withdrawing groups.

4. The process according to claim 3, wherein said one or more electron-withdrawing groups are in a vicinal position with respect to the nitrile group.

5. The process according to any one of claims 3-4, wherein said one or more electron-withdrawing groups are selected from a nitro group, a halogen, such as chlorine, fluorine, bromine, iodine, or a phenyl.

6. The process according to any one of claims 1-5, wherein the arsenous acid generator is selected from the group consisting of $As_2O_3$ or an arsenite, for example an arsenite with an alkali or alkaline earth metal.

7. The process according to any one of claims 1-6, wherein the Pd(II) generator is selected in the group consisting of a Pd(IV) compound, a Pd(0) compound, or Pd(0).

8. The process according to any one of claims 1-7, wherein the palladium(II) compound is selected from the group consisting of a salt of Pd+2 with the formula $PdX_2$, wherein X is selected in the group consisting of Cl, Br, I, or NOs; $Pd(OTf)_2(dppp)$; $Pd(OTf)_2(dppe)$; palladium acetate; or $Pd(dppf)Cl_2$.

9. The process according to any one of claims 1-8, wherein the first compound and the second compound of the catalytic system are added in a 1:1 ratio.

10. The process according to any one of claims 1-9, said process further comprising the following step:
    d) isolating the amide produced in step c) from the reaction environment.

11. The process according to claim 10, wherein step d) is carried out using a technique selected in the group consisting of column chromatography, crystallisation, precipitation, sublimation, or distillation.

12. The process according to any one of claims 1-11, said process further comprising the following step:
    e) regenerating the catalytic system.

FIG. 1

FIG. 2

*Acetamide CDCl3*

—1.9695

—5.8727
—6.0954

—7.2592

3.0000

2.1808

**FIG.    3**

**FIG. 4**

Acetamide CDCl3

—173.3770

77.4150
77.1604
76.9062

—22.7225

200     150     100     50     0

FIG. 5

FIG. 6

methacrylamide DMSO-d6

# FIG. 7

methacrylamide DMSO-d6

—19.1813

—120.0133

—140.3620

—169.8224

FIG.8

FIG. 9

**FIG. 10**

benzamide DMSO-d6

7.4224
7.4370
7.4524
7.4665
7.4872
7.4894
7.5022
7.5165
7.8881
7.9039
8.0155

2.4971

3.4346

2.9213

1.0150

2.9213

1.0209
2.0000

# FIG. 11

benzamide DMSO-d6

128.0033
128.7441
131.7678
134.7763

168.5445

**FIG. 12**

FIG. 13

**FIG. 14**

2-hydroxybenzamide DMSO-d6

—172.7231
—161.6807
—134.5842
—128.6469
—118.8825
—117.9621
—114.9085

200    150    100    50    0

*p-(dimethylamino)benzamide DMSO-d6*

**FIG. 15**

p-(dimethylamino)benzamide DMSO-d6

40.2548

111.2343

121.4859

129.4461

152.6525

168.4707

FIG. 16

*p-bromobenzamide DMSO-d6*

7.4809
7.6402
7.6570
7.8126
7.8294
8.0634

3.3911

2.4995

2.0000
0.9653

0.9921
2.0346

**FIG. 17**

p-bromobenzamide DMSO-d6

167.5321

133.9185
131.7740
130.1542
125.5861

**FIG. 18**

FIG. 19

*p-nitrobenzamide DMSO-d6*

123.9509
129.4358
140.4942
149.5671
166.7762

FIG. 20

FIG. 21

FIG. 22

4-cyanobenzamide DMSO-d6

—2.4970

—3.3364

7.9390
7.9561
8.0037
8.0208
8.1706
8.2105

2.0000
1.6374
2.1268

FIG. 23

4-cyanobenzamide DMSO-d6

110.8461
114.1782
128.7985
132.9425
138.8334
166.9697

FIG. 24

*4-carbamoyl-1-methylpyridin-1-ium nitrate DMSO-d6*

FIG. 25

4-carbamoyl-1-methylpyridin-1-ium nitrate DMSO-d6

40.0177

48.5375

125.9553

147.0792
148.4031

163.9879

0

50

100

150

200

**FIG. 26**

Europäisches
Patentamt

European
Patent Office

Office européen
des brevets

## EUROPEAN SEARCH REPORT

Application Number

EP 24 15 4384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAO WANG ET AL: "Preparation of benzamide by hydration of benzonitrile with palladium acetate and phenylarsonic acid as synergistic catalyst", CHINESE JOURNAL OF INORGANIC CHEMISTRY, vol. 35, no. 9, 1 January 2019 (2019-01-01), pages 1619-1622, XP093074949, * table 1 * * abstract * | 1-12 | INV. C07C231/06 C07C233/05 C07C233/09 C07C233/11 C07C233/65 C07D213/00 |
| X | JP S52 89619 A (NIPPON KAYAKU KK) 27 July 1977 (1977-07-27) * paragraph [0002] - paragraph [0003]; tables 1-3 * | 1-12 | |
| Y | SICHENG ZHANG ET AL: "Efficient Bimetallic Catalysis of Nitrile Hydration to Amides with a Simple Pd(OAc)2/Lewis Acid Catalyst at Ambient Temperature", EUROPEAN JOURNAL OF ORGANIC CHEMISTRY, WILEY-VCH, DE, vol. 2017, no. 14, 11 April 2017 (2017-04-11), pages 1870-1875, XP072110493, ISSN: 1434-193X, DOI: 10.1002/EJOC.201601495 * figures Scheme 2,3 * * tables 3,4 * | 1-12 | TECHNICAL FIELDS SEARCHED (IPC) C07C |
| Y | EP 0 078 768 A1 (CIBA GEIGY AG [CH]) 11 May 1983 (1983-05-11) * page 7, paragraph 2 - page 8, paragraph 1 * | 1-12 | |

The present search report has been drawn up for all claims

| Place of search | Date of completion of the search | Examiner |
|---|---|---|
| The Hague | 21 June 2024 | Kövecs, Monika |

EPO FORM 1503 03.82 (P04C01)

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

## ANNEX TO THE EUROPEAN SEARCH REPORT
## ON EUROPEAN PATENT APPLICATION NO.

EP 24 15 4384

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2024

| Patent document cited in search report | | Publication date | Patent family member(s) | | Publication date |
|---|---|---|---|---|---|
| JP S5289619 | A | 27-07-1977 | JP | S5289619 A | 27-07-1977 |
| | | | JP | S5814429 B2 | 18-03-1983 |
| EP 0078768 | A1 | 11-05-1983 | BR | 8206388 A | 27-09-1983 |
| | | | CH | 654286 A5 | 14-02-1986 |
| | | | EP | 0078768 A1 | 11-05-1983 |
| | | | ES | 8404669 A1 | 16-05-1984 |
| | | | JP | S5888328 A | 26-05-1983 |
| | | | US | 4564479 A | 14-01-1986 |

EPO FORM P0459

For more details about this annex : see Official Journal of the European Patent Office, No. 12/82

## REFERENCES CITED IN THE DESCRIPTION

### Patent documents cited in the description

- JP 5289619 B
- JP S5289619 B

- US 3312620 A

### Non-patent literature cited in the description

- **TAO WANG et al.** Preparation of benzamide by hydration of benzonitrile with palladium acetate and phenylarsonic acid as synergistic catalyst. *Chinese Journal of Inorganic Chemistry,* 2019, vol. 35 (9
- **J. E. REIMANN ; R. U. BYERRUM.** The Chemistry of Amides. John Wiley & Sons Ltd, 1970, 601-684
- **C. THE BERRE ; P. SERP ; P. KALCK ; G. P. TORRENCE.** Ullmann's Encyclopedia of Industrial Chemistry, Acetic Acid. Wiley-VCH, 2014, vol. 1, 231
- **S. KHALKAR ; D. BHOWMICK ; A. PRATAP.** *J Oleo Sci.,* 2013, vol. 62 (11), 901-904
- **H. LOW ; J. E. LAUCK.** *Amide Lubricants,* 1967
- **F. MESSA ; S. PERRONE ; M. CAPUA ; F. TOLOMEO ; L. TROISI ; V. CAPRIATI ; A. SALOMONE.** *Chem. Commun.,* 2018, vol. 54, 8100-8103
- **C. A. G. N. MONTALBETTI ; V. FALQUE.** *Tetrahedron,* 2005, vol. 61, 10827-10852
- **J. N. HAHLADAKIS ; C. A. VELIS ; R. WEBER ; E. LACOVIDOU ; P. PURNEL.** *J. Haz. Mat.,* 2018, vol. 344, 179-199
- **T. OJEDA.** Polymer Science. IntechOpen, 2013

- **V. Y. KUKUSHIN ; A. J. L. POMBEIRO.** *Inorg. Chim. Acta,* 2005, vol. 358, 1-21
- **Z. CHENG ; Y. XIA ; Z. ZHOU.** *Front. Bioeng. Biotechnol.,* 2020, vol. 8, 352
- **A. R. ALCÁNTARA ; P. DOMÍNGUEZ DE MARIA ; J. A. LITTLECHILD ; M. SCHÜRMANN ; R. A. SHELDON ; R. WOHLGEMUTH.** *ChemSusChem,* 2022, e202102709
- **M. C. BRYAN ; P. J. DUNN ; D. ENTWISTLE ; F. GALLOU ; S. G. KOENIG ; J. D. HAYLER ; M. R. HICKEY ; S. HUGHES ; M. E. KOPACH ; G. MOINE.** *Green Chem.,* 2018, vol. 20, 5082-5103
- **R. GARCÍA-ÁLVAREZ ; J. FRANCOS ; E. TOMÁS-MENDIVIL ; P. CROCHET ; V. CADIERNO.** *J. Organomet. Chem.,* 2014, vol. 771, 93-104
- **E. TOMÁS-MENDIVIL ; R. GARCÍA-ÁLVAREZ ; C. VIDAL ; P. CROCHET ; V. CADIERNO.** *ACS Catal.,* 2014, vol. 4, 1901-1910
- **M. N. SOKOLOV ; A. V. VIROVETS ; D. N. DYBTSEV ; E. V. CHUBAROVA ; V. P. FEDIN ; D. FENSKE.** *Inorg. Chem.,* 2001, vol. 40, 4816-1817
- **D. U. MIODRAGOVIC ; J. A. QUENTZEL ; J. W. KURUTZ ; C. L. STERN ; R. W. AHN ; I. KANDELA ; A. MAZAR ; T. V. O'HALLORAN.** *Angew. Chem. Int. Ed.,* 2013, vol. 52, 1-5